# EUROPÄISCHE PATENTSCHRIFT

(11) **EP 1 530 555 B1**
(45) Veröffentlichungstag und Bekanntmachung des Hinweises auf die Patenterteilung: **16.01.2008**
(21) Anmeldenummer: 03771072.0
(22) Anmeldetag: 23.07.2003
(51) Int. Cl.: C07C 7/08, C07C 11/167

(54) **VERFAHREN ZUR AUFARBEITUNG VON ROH-1,3-BUTADIEN**
METHOD FOR WORKING UP CRUDE 1,3-BUTADIENE
PROCEDE POUR RETRAITER DU 1,3-BUTADIENE BRUT

(30) Priorität: 24.07.2002 DE 10233621
(43) Veröffentlichungstag der Anmeldung: 18.05.2005
(73) Patentinhaber: BASF Aktiengesellschaft, 67056 Ludwigshafen (DE)
(72) Erfinder: HILL, Thomas, 67051 Ludwigshafen (DE); KINDLER, Klaus, 67376 Harthausen (DE); HEIDA, Bernd, 67158 Ellerstadt (DE)
(74) Vertreter: Hörschler, Wolfram Johannes
(86) Internationale Anmeldenummer: PCT/EP2003/008044
(87) Internationale Veröffentlichungsnummer: WO 2004/011407

(56) Entgegenhaltungen:
- US-A- 4 049 742
- US-B1- 6 337 429

## Beschreibung

Die Erfindung betrifft ein Verfahren zur Aufarbeitung eines Gemisches von Kohlenwasserstoffen, das durch Extraktivdestillation aus einem C₄-Schnitt erhalten wurde.

Der Begriff C₄-Schnitt bezeichnet Gemische von Kohlenwasserstoffen mit überwiegend 4 Kohlenstoffatomen pro Molekül. C₄-Schnitte werden beispielsweise bei der Herstellung von Ethylen und/oder Propylen durch thermisches Spalten, üblicherweise in Steamcrackern oder FCC-Crackern (Fluidized Catalytic Cracking) einer Petroleumfraktion, wie verflüssigtes Petroleumgas, Leichtbenzin oder Gasöl, erhalten. Weiterhin werden C₄-Schnitte bei der katalytischen Dehydrierung von n-Butan und/oder n-Buten erhalten. C₄-Schnitte enthalten in der Regel Butane, n-Buten, Isobuten, 1,3-Butadien, daneben geringe Mengen an sonstigen Kohlenwasserstoffen, darunter Butine, insbesondere 1-Butin (Ethylacetylen) und Butenin (Vinylacetylen). Dabei beträgt der 1,3-Butadiengehalt von C₄-Schnitten aus Steamcrackern im allgemeinen 10 bis 80 Gew.-%, vorzugsweise 20 bis 70 Gew.-%, insbesondere 30 bis 60 Gew.-%, während der Gehalt an Vinylacetylen und Ethylacetylen im allgemeinen 5 Gew.-% nicht übersteigt.

Die Auftrennung von C₄-Schnitten ist wegen der geringen Unterschiede in den relativen Flüchtigkeiten der Komponenten ein kompliziertes destillationstechnisches Problem. Daher wird die Auftrennung durch eine sogenannte Extraktivdestillation durchgeführt, d. h. eine Destillation unter Zugabe eines selektiven Lösungsmittels (auch als Extraktionsmittel bezeichnet), das einen höheren Siedepunkt als das aufzutrennende Gemisch aufweist und das die Unterschiede in den relativen Flüchtigkeiten der aufzutrennenden Komponenten erhöht.

Es sind eine Vielzahl von Verfahren zur Auftrennung von C₄-Schnitten mittels Extraktivdestillation unter Verwendung von selektiven Lösungsmitteln bekannt. Ihnen ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, häufig im Bereich von 20 bis 80°C und bei moderaten Drücken, häufig bei Normaldruck bis 6 bar, das selektive Lösungsmittel mit den Komponenten aus dem C₄-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck gegenüber dem ersten Verfahrensschritt, in einem oder mehreren weiteren Verfahrensschritten die Komponenten fraktioniert aus dem selektiven Lösungsmittel freigesetzt.

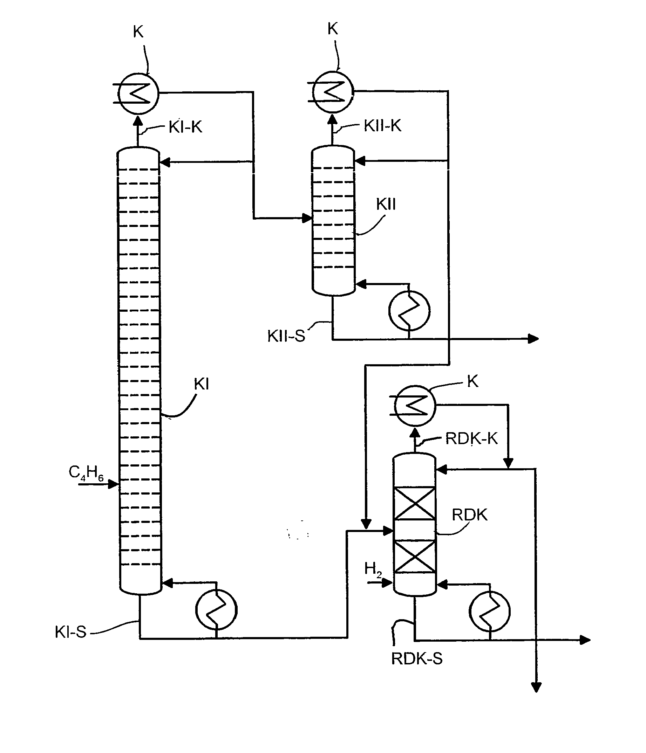

Häufig wird die Extraktivdestillation von C₄-Schnitten in der Weise gefahren, dass die Komponenten des C₄-Schnittes, für die das selektive Lösungsmittel eine geringere Affinität als für 1,3-Butadien hat, insbesondere die Butane und die Butene, im Wesentlichen in der Gasphase verbleiben, wogegen 1,3-Butadien sowie weitere Kohlenwasserstoffe, für die das selektive Lösungsmittel eine höhere Affinität als für 1,3-Butadien hat, vom selektiven Lösungsmittel im Wesentlichen vollständig absorbiert werden. Die Gasphase wird als Kopfstrom abgezogen und häufig als Raffinat 1 bezeichnet. Ein derartiges Verfahren ist beispielsweise in der DE-A 198 188 10 beschrieben, wobei das Raffinat 1 der in den Figuren 1 und 2 mit Gbc bezeichnete Kopfstrom der Extraktivdestillationskolonne E I ist.

Die Aufarbeitung des mit 1,3-Butadien sowie mit weiteren Kohlenwasserstoffen, für die das selektive Lösungsmittel eine höhere Affinität als für 1,3-Butadien hat beladenen selektiven Lösungsmittels erfolgt in der Regel durch fraktionierte Desorption, wobei die im selektiven Lösungsmittel absorbierten Kohlenwasserstoffe in der umgekehrten Reihenfolge ihrer Affinität zum selektiven Lösungsmittel desorbiert werden.

Ein derartiges Verfahren ist beispielsweise in DE-A 198 188 10 beschrieben, wonach das selektive Lösungsmittel, das mit 1,3-Butadien und mit sonstigen C₄-Kohlenwasserstoffen beladen ist, und als sogenannte Extraktionslösung ad bezeichnet ist, in einer Verfahrensstufe 3 in eine Desorptionszone mit gegenüber der Extraktionszone vermindertem Druck und/oder erhöhter Temperatur überführt und dabei aus der Extraktionslösung ad 1,3-Butadien desorbiert wird, wobei der Hauptteil der sonstigen C₄-Kohlenwasserstoffe in der flüssigen Phase verbleibt. Dabei werden zwei getrennte Ströme abgezogen, und zwar 1,3-Butadien als Roh-1,3-Butadien-Strom und das mit sonstigen C₄-Kohlenwasserstoffen beladene selektive Lösungsmittel als Extraktionslösung d. Aus der Extraktionslösung d werden schließlich in einer zweiten Desorptionszone mit gegenüber der ersten Desorptionszone vermindertem Druck und/oder erhöhter Temperatur und mit einem Druck- und/oder Temperaturgradienten noch darin verbliebenes 1,3-Butadien und die sonstigen C₄-Kohlenwasserstoffe mindestens als zwei getrennte Fraktionen fraktioniert desorbiert.

Nach herrschender Meinung war es bislang nicht möglich, die Acetylene und das 1,2-Butadien aus dem Roh-1,3-Butadien mit vertretbarem wirtschaftlichem Aufwand destillativ abzutrennen. Besonders problematisch waren hierbei die geringen Unterschiede in den relativen Flüchtigkeiten sowie die hohe Reaktivität der den Roh-1,3-Butadien-Strom bildenden Komponenten.

Demgegenüber war es Aufgabe der Erfindung, ein Verfahren zur Verfügung zu stellen, das mit vertretbarem wirtschaftlichem Aufwand die destillative Abtrennung von Acetylenen und 1,2-Butadien aus einem Roh-1,3-Butadien-Strom ermöglicht und dabei gleichzeitig eine sichere Verfahrensführung gewährleistet. Das erfindungsgemäße Verfahren ermöglicht somit eine destillative Aufarbeitung von Roh-1,3-Butadien-Strömen ohne den aufwändigen vorgeschalteten Verfahrensschritt einer Abtrennung der Acetylene durch Extraktivdestillation mit einem selektiven Lösungsmittel.

Die Lösung besteht in einem kontinuierlichen Verfahren zur Auftrennung eines Gemisches von Kohlenwasserstoffen, das durch Extraktivdestillation eines C₄-Schnittes mit einem selektiven Lösungsmittel erhalten wurde, und das die Kohlenwasserstoffe aus dem C₄-Schnitt umfasst, die im selektiven Lösungsmittel besser löslich sind als die Butane und die Butene , das dadurch gekennzeichnet ist, dass das Gemisch einer ersten Destillationskolonne zugeführt wird, worin es in einen Kopfstrom aufgetrennt wird, umfassend 1,3-Butadien, Propin, gegebenenfalls weitere Leichtsieder und gegebenenfalls Wasser sowie in einen Sumpfstrom umfassend 1,3-Butadien, 1,2-Butadien, Acetylene sowie gegebenenfalls weitere Schwersieder, wobei der Anteil des 1,3-Butadiens im Sumpfstrom der Destillationskolonne dergestalt geregelt wird, dass er mindestens so hoch ist, dass er die Acetylene außerhalb des selbstzersetzungsgefährdeten Bereiches verdünnt und dass der Kopfstrom der ersten Destillationskolonne einer zweiten Destillationskolonne zugeführt und in der zweiten Destillationskolonne in einen Kopfstrom, umfassend Propin, gegebenenfalls weitere Leichtsieder und gegebenenfalls Wasser und einen Sumpfstrom, umfassend Rein-1,3-Butadien mit einem Gehalt von mindestens 99 Gew.-% Butadien, aufgetrennt wird.

Erfindungsgemäß wird somit ein Roh-1,3-Butadien-Strom in einer Destillationskolonne einer bezüglich 1,3-Butadien unscharfen destillativen Auftrennung unterworfen, wobei die Acetylene und 1,2-Butadien als Sumpfstrom abgezogen werden, der mit 1,3-Butadien außerhalb des selbstzersetzungsgefährdeten Bereiches verdünnt ist. Im Übrigen wird Butadien, gemeinsam mit Propin, gegebenenfalls weiteren Leichtsiedern und gegebenenfalls Wasser als Kopfstrom der Destillationskolonne abgezogen.

Der Kopfstrom der Destillationskolonne wird bevorzugt in einem Kondensator am Kolonnenkopf kondensiert, teilweise als Rücklauf erneut auf die Kolonne aufgegeben und im Übrigen einer zweiten Destillationskolonne zugeführt, und darin in einen Kopfstrom, der Propin und gegebenenfalls weitere Leichtsieder umfasst und in einen Sumpfstrom, umfassend Rein-1,3-Butadien, aufgetrennt.

In beiden vorstehend beschriebenen Destillationskolonnen können grundsätzlich alle für Butadien-Destillationen üblichen trennwirksamen Einbauten eingesetzt werden. Weil sie sich leichter reinigen lassen, sind Böden besonders geeignet.

Die Zusammensetzung des Roh-1,3-Butadienstromes ist abhängig von der Zusammensetzung des C₄-Schnittes, der der Extraktivdestillation zugeführt wurde und umfasst in der Regel die gesamten Acetylene, das gesamte 1,2-Butadien, 30 bis 70 % des cis-2-Butens sowie mindestens 99 % des 1,3-Butadiens aus dem C₄-Schnitt.

Dabei werden vorliegend die niedriger als 1,3-Butadien siedenden Kohlenwasserstoffe als Leichtsieder und die höher als 1,3-Butadien siedenden Kohlenwasserstoffe als Schwersieder bezeichnet. Ein typischer Leichtsieder ist Propin, Schwersieder sind überwiegend Kohlenwasserstoffe mit einer Dreifachbindung, im Folgenden als Acetylene bezeichnet, insbesondere 1-Butin (Ethylacetylen) und Butenin (Vinylacetylen).

Der vorliegend in Verbindung mit der Zusammensetzung von bei der destillativen Aufarbeitung erhaltenen Strömen verwendete Begriff "gegebenenfalls" bedeutet, dass die danach aufgeführten Komponenten, je nach konkreter Verfahrensführung, insbesondere je nach Zusammensetzung des eingesetzten C₄-Schnittes, des eingesetzten Lösungsmittels und/oder eingesetzter Hilfsstoffe in den jeweiligen Strömen vorhanden sein können.

Die destillative Abtrennung der Acetylene und des 1,2-Butadiens aus dem Roh-1,3-Butadien ist aufgrund der hohen Reaktivität derselben sowie aufgrund der geringen Unterschiede in relativen Flüchtigkeiten der den Roh-1,3-Butadien-Strom bildenden Komponenten ein kompliziertes destillationstechnisches Problem. Es wurde jedoch überraschend gefunden, dass die destillative Abtrennung der Acetylene und des 1,2-Butadiens mit vertretbarem energetischem Aufwand möglich ist und dabei gleichzeitig eine sichere Verfahrensführung gewährleistet werden kann, indem die Acetylene und das 1,2-Butadien als Sumpfstrom aus einer Destillationskolonne abgezogen werden, und dabei mit 1,3-Butadien au-ßerhalb des selbstzersetzungsgefährdeten Bereiches verdünnt werden. Hierfür ist in der Regel eine Verdünnung des Sumpfstromes auf unterhalb von 30 Mol-%, Acetylene ausreichend. C4-Schnitte weisen in der Regel Zusammensetzungen in Gew-% in den nachstehenden Bereichen auf:

| | |
|---|---|
| 1,3-Butadien | 10 bis 80 |
| Butene | 10 bis 60 |
| Butane | 5 bis 40 |
| sonstige C₄-Kohlenwasserstoffe und | 0,1 bis 5 |
| sonstige Kohlenwasserstoffe, insbesondere | |
| C₃- und C₅-Kohlenwasserstoffe | 0 bis maximal 5. |

Der Begriff Rein-1,3-Butadien bezeichnet vorliegend einen Strom mit einem Gehalt von mindestens 99 Gew.-% 1,3-Butadien, bevorzugt von mindestens 99,6 Gew.-% 1,3-Butadien, Rest Verunreinigungen, insbesondere 1,2-Butadien und cis-2-Buten.

In einer bevorzugten Verfahrensalternative werden der Sumpfstrom aus der ersten Destillationskolonne und der Kopfstrom aus der zweiten Destillationskolonne einer Reaktivdestillationskolonne zugeführt, in der in heterogener Katalyse mit Wasserstoff eine Selektivhydrierung der Dreifachbindungen aufweisenden Kohlenwasserstoffe zu Doppelbindungen aufweisenden Kohlenwasserstoffen durchgeführt wird, bei Teilumsatz der Acetylene, unter Erhalt eines Kopfstromes, umfassend 1,3-Butadien, Butane, Butene sowie nicht-hydrierte Kohlenwasserstoffe mit Dreifachbindungen und einen Schwersieder umfassenden Sumpfstrom, der ausgeschleust wird.

Insbesondere wird Vinylacetylen zum Wertprodukt 1,3-Butadien selektiv hydriert.

Der Kopfstrom der Reaktivdestillationskolonne oder ein Teilstrom desselben kann bevorzugt in die Extraktivdestillationskolonne rezykliert werden. Es ist jedoch auch möglich, den Kopfstrom der Reaktivdestillationskolonne oder einen Teilstrom desselben aus der Anlage abzuziehen, anderweitig weiter zu verarbeiten, beispielsweise einem Cracker-Feed beizumischen, oder zu verbrennen.

Die bevorzugte Verfahrensweise mit der Extraktivdestillation nachgeschalteter Selektivhydrierung der Acetylene ist verfahrenstechnisch insbesondere bezüglich der Auswahlmöglichkeiten für den Katalysator vorteilhaft, da die Selektivhydrierung in einer Verfahrensstufe durchgeführt wird, in der praktisch kein selektives Lösungsmittel mehr im Reaktionsgemisch vorliegt. Würde die Selektivhydrierung dagegen, wie in bekannten Verfahren, in der Extraktivdestillationskolonne und somit in Gegenwart des selektiven Lösungsmittels durchgeführt werden, so wäre die Auswahl des Katalysators erheblich durch das selektive Lösungsmittel eingeschränkt, das die Hydrierung unselektiver machen kann. In der der Extraktivdestillation nachgeschalteten Selektivhydrierung gibt es dagegen keine derartigen Einschränkungen bezüglich der Katalysatorauswahl.

Die Erfindung wird im Folgenden anhand einer Zeichnung sowie eines Ausführungsbeispiels näher erläutert.

Figur 1 zeigt die schematische Darstellung einer Anlage zur destillativen Auftrennung eines Roh-1,3-Butadien-Stromes.

Ein Roh-1,3-Butadien-Strom C₄H₆ bezeichnet, wird einer ersten Destillationskolonne K I zugeführt, und in derselben in einen Kopfstrom K I-K und einen Sumpfstrom K I-S aufgetrennt. Der Kopfstrom K I-K wird in einem Kondensator K am Kolonnenkopf kondensiert, teilweise als Rücklauf wieder auf die Kolonne aufgegeben, und im Übrigen abgezogen und einer zweiten Destillationskolonne K II zugeführt. Der Sumpfstrom K I-S wird abgezogen und einer Reaktivdestillationskolonne RDK zugeführt.

In der zweiten Destillationskolonne K II erfolgt eine Auftrennung in einen Kopfstrom K II-K, der in einem Kondensator K kondensiert, teilweise als Rücklauf wieder auf die Kolonne aufgegeben und im Übrigen ebenfalls in die Reaktivdestillationskolonne RDK geführt wird. Der Sumpfstrom K II-S der zweiten Destillationskolonne K II wird als Rein-1,3-Butadien-Strom abgezogen.

In der Reaktivdestillationskolonne RDK werden durch Selektivhydrierung mit Wasserstoff in Gegenwart eines heterogenen Katalysators die Dreifachbindungen aufweisenden Kohlenwasserstoffe zu Doppelbindungen aufweisenden Kohlenwasserstoffen hydriert. Es wird ein Kopfstrom RDK-K abgezogen, in einem Kondensator K kondensiert, teilweise erneut auf der Reaktivdestillationskolonne RDK aufgegeben und im Übrigen vorzugsweise, wie in der Fig. dargestellt, in die Extraktivdestillationskolonne rezykliert.

Der Sumpfstrom der Reaktivdestillationskolonne, Strom RDK-S, der überwiegend Schwersieder enthält, wird aus der Anlage ausgeschleust und vorzugsweise verbrannt.

### Beispiel: Destillative Aufarbeitung von Roh-1,3-Butadien

Ein Roh-1,3-Butadienstrom C₄H₆, der durch Extraktivdestillation aus einem C₄-Schnitt gewonnen wurde, wurde einer Destillationskolonne mit 80 theoretischen Trennstufen auf der 25. Stufe, bei Zählung der Trennstufen von unten nach oben, zugeführt. Der Roh-1,3-Butadienstrom C₄H₆ hatte die folgende Zusammensetzung in Gew.-%:

| | |
|---|---|
| Propin | 0,11 |
| 1,3-Butadien | 98,58 |
| 1,2-Butadien | 0,30 |
| 1-Butin | 0,30 |
| Vinylacetylen | 0,56 |
| Wasser | 0,15. |

Er wurde in der ersten Destillationskolonne K I in einen Kopfstrom K I-K aufgetrennt, mit nachstehender Zusammensetzung in Gew.-%:

| | |
|---|---|
| Propin | 0,11 |
| 1,3-Butadien | 99,73 |
| Wasser | 0,16 |

sowie in einen Sumpfstrom K I-S, mit nachstehender Zusammensetzung in Gew.-%:

| | |
|---|---|
| cis-Buten-2 | 0,52 |
| 1,3-Butadien | 40,0 |
| 1,2-Butadien | 15,1 |
| 1-Butin | 13,75 |
| Vinylacetylen | 29,17 |
| 3-Methylbuten-1 | 0,98 |
| 2-Methylbuten-2 | 0,48. |

Der Kopfstrom K I-K der ersten Destillationskolonne K I wurde in einen Abzug (1/7 des Kopfstromes K I-K) und in einen Rücklauf (6/7 des Kopfstromes K I-K) aufgeteilt. Der Abzug wurde einer zweiten Destillationskolonne K II mit 25 theoretischen Trennstufen, auf die 14. Trennstufe zugeführt, und in einen Kopfstrom K II-K mit folgender Zusammensetzung in Gew.-%:

| | |
|---|---|
| Propin | 79,52 |
| 1,3-Butadien | 20,0 und |
| Wasser | 0,48 |

sowie einen Sumpfstrom K II-S, umfassend Rein-1,3-Butadien, mit einem Gehalt an 1,3-Butadien von 99,99 %, aufgetrennt. Der Sumpfstrom K II-S wurde als Wertprodukt abgezogen.

Gegenüber einem bekannten Verfahren zur Gewinnung von 1,3-Butadien aus einem C₄-Schnitt, mit Abtrennung der Acetylene von 1,3-Butadien durch Extraktivdestillation mit einem selektiven Lösungsmittel wurde durch das vorliegende Verfahren eine Energieeinsparung von ca. 9 % erreicht. Darüber hinaus wurde die Anlage durch Einsparung der Kolonne für die Abtrennung der Acetylene von 1,3-Butadien durch Extraktivdestillation vereinfacht, mit entsprechender Reduzierung der Investitionskosten und des Platzbedarfes.

## Patentansprüche

1. Kontinuierliches Verfahren zur Auftrennung eines Gemisches von Kohlenwasserstoffen, das durch Extraktivdestillation eines C₄-Schnittes (C₄) mit einem selektiven Lösungsmittel (LM) erhalten wurde, und das die Kohlenwasserstoffe aus dem C₄-Schnitt (C₄) umfasst, die im selektiven Lösungsmittel (LM) besser löslich sind als die Butane und die Butene, **dadurch gekennzeichnet, dass** das Gemisch einer ersten Destillationskolonne (K I) zugeführt wird, worin es in einen Kopfstrom (K I-K) aufgetrennt wird, umfassend 1,3-Butadien, Propin, gegebenenfalls weitere Leichtsieder und gegebenenfalls Wasser sowie in einen Sumpfstrom (K I-S), umfassend 1,3-Butadien, 1,2-Butadien, Acetylene sowie gegebenenfalls weitere Schwersieder, wobei der Anteil des 1,3-Butadiens im Sumpfstrom (K I-S) der Destillationskolonne (K I) dergestalt geregelt wird, dass er mindestens so hoch ist, dass er die Acetylene au-ßerhalb des selbstzersetzungsgefährdeten Bereiches verdünnt und dass der Kopfstrom (K I - K) der ersten Destillationskolonne (K I) einer zweiten Destillationskolonne (K II) zugeführt und in der zweiten Destillationskolonne (K II) in einen Kopfstrom (K II-K), umfassend Propin, gegebenenfalls weitere Leichtsieder und gegebenenfalls Wasser und einen Sumpfstrom (K II-S), umfassend Rein-1,3-Butadien mit einem Gehalt von mindestens 99 Gew.-% Butadien, aufgetrennt wird.

2. Verfahren nach Anspruch 1, **dadurch gekennzeichnet, dass** der Anteil des 1,3-Butadiens im Sumpfstrom (K I-S) der Destillationskolonne (K I) dergestalt geregelt wird, dass der Anteil der Acetylene im Sumpfstrom (K I-S) kleiner als 30 Mol.-% ist.

3. Verfahren nach Anspruch 1, **dadurch gekennzeichnet, dass** der Sumpfstrom aus der ersten Destillationskolonne (K I) und der Kopfstrom aus der zweiten Destillationskolonne (K II) einer Reaktivdestillationskolonne (RDK) zugeführt werden, in der in heterogener Katalyse mit Wasserstoff eine Selektivhydrierung der Dreifachbindungen aufweisenden Kohlenwasserstoffe zu Doppelbindungen aufweisenden Kohlenwasserstoffen durchgeführt wird, bei Teilumsatz der Acetylene, unter Erhalt eines Kopfstromes, umfassend 1,3-Butadien, Butane, Butene sowie nicht-hydrierte Kohlenwasserstoffe mit Dreifachbindungen und einen Schwersieder umfassenden Sumpfstrom, der ausgeschleust wird.

4. Verfahren nach einem der Ansprüche 1 bis 3, **dadurch gekennzeichnet, dass** der Kopfstrom (RDK-K) aus der Reaktivdestillationskolonne (RDK) oder ein Teilstrom desselben in die Extraktivdestillation rezykliert wird.

## Claims

1. A continuous process for separating a mixture of hydrocarbons which has been obtained by extractive distillation of a C₄ fraction (C₄) using a selective solvent (LM) and comprises the hydrocarbons from the C₄ fraction (C₄) which are more readily soluble in the selective solvent (LM) than are the butanes and the butenes, which comprises feeding the mixture into a first distillation column (K I) in which it is separated into a stream (K I-K) which is taken off at the top and comprises 1,3-butadiene, propyne, possibly further low boilers and possibly water and a bottom stream (K I-S) comprising 1,3-butadiene, 1,2-butadiene, acetylenes and possibly further high boilers, with the proportion of 1,3-butadiene in the bottom stream (K I-S) from the distillation column (K I) being regulated in such a way that it is at least sufficiently high to dilute the acetylenes to outside the range in which there is a risk of spontaneous decomposition, and passing the stream (K I-K) taken off from the top of the first distillation column (K I) to a second distillation column (K II) and separating it into a stream (K II-K) which is taken off at the top and comprises propyne, possibly further low boilers and possibly water and a bottom stream (K II-S) comprising pure 1,3-butadiene having a butadiene content of at least 99% by weight in the second distillation column (K II).

2. The process according to claim 1, wherein the proportion of 1,3-butadiene in the bottom stream (K I-S) from the distillation column (K I) is regulated in such a way that the proportion of acetylenes in the bottom stream (K I-S) is less than 30 mol%.

3. The process according to claim 1, wherein the bottom stream from the first distillation column (K I) and the stream from the top of the second distillation column (K II) are fed to a reactive distillation column (RDK) in which a selective hydrogenation of the hydrocarbons containing triple bonds to hydrocarbons containing double bonds is carried out by means of hydrogen in the presence of a heterogeneous catalyst, with partial conversion of the acetylenes, to give a stream comprising 1,3-butadiene, butanes, butenes and hydrocarbons containing triple bonds which have not been hydrogenated at the top and a bottom stream comprising high boilers which is discharged.

4. The process according to any of claims 1 to 3, wherein the stream (RDK-K) taken off at the top of the reactive distillation column (RDK) or a substream thereof is recycled to the extractive distillation

## Revendications

1. Procédé continu pour la séparation d'un mélange d'hydrocarbures obtenu par distillation extractive d'une coupe en C₄ (C₄) avec un solvant sélectif (LM), et qui contient les hydrocarbures de la coupe en C₄ (C₄) qui ont une solubilité supérieure dans le solvant sélectif (LM) à celle des butanes et des butènes, **caractérisé en ce que** le mélange est envoyé à une première colonne de distillation (KYI) dans laquelle il est séparé en un courant de tête (KI-K) contenant du 1,3-butadiène, du propyne, le cas échéant d'autres constituants à bas point d'ébullition et le cas échéant de l'eau, et un courant de fond (KI-S) contenant du 1,3-butadiène, du 1,2 butadiène, de l'acétylène, et le cas échéant d'autres constituants à haut point d'ébullition, où la fraction du 1,3-butadiène dans le courant de fond (KI-S) de la colonne de distillation (KI) est régulée de manière à être au moins suffisamment élevée pour que l'acétylène soit dilué hors de la plage à risque de décomposition spontanée et **en ce que** le courant de tête (KI-K) de la première colonne de distillation (KI) est envoyé à une deuxième colonne de distillation (KII) et, dans la deuxième colonne de distillation (KII), est séparé en un courant de tête (KII-K) contenant du propyne, le cas échéant d'autres constituants à bas point d'ébullition et le cas échéant de l'eau, et un courant de fond (KII-S) contenant du 1,3-butadiène pur ayant une teneur d'au moins 99 % en poids de butadiène.

2. Procédé suivant la revendication 1, **caractérisé en ce que** la fraction de 1,3-butadiène dans le courant de fond (KI-S) de la colonne de distillation (KYI) est régulée de manière à ce que la fraction d'acétylène dans le courant de fond (KI-S) soit de moins de 30 % molaires.

3. Procédé suivant la revendication 1, **caractérisé en ce que** le courant de fond de la première colonne de distillation (KI) et le courant de tête de la deuxième colonne de distillation (KII) sont envoyés dans une colonne de distillation réactive (RDK) dans laquelle est entreprise, avec catalyse hétérogène et avec de l'hydrogène, une hydrogénation sélective des hydrocarbures présentant des triples liaisons en hydrocarbures présentant des doubles liaisons, par réaction partielle de l'acétylène, avec obtention d'un courant de tête contenant du 1,3-butadiène, des butanes, des butènes, ainsi que des hydrocarbures non hydrogénés ayant des triples liaisons, et un courant de fond contenant des constituants à haut point d'ébullition, qui est éclusé.

4. Procédé suivant l'une quelconque des revendications 1 à 3, **caractérisé en ce que** le courant de tête (RDK-K) de la colonne de distillation réactive (RDK) ou un courant partiel de celui-ci est recyclé dans la colonne de distillation extractive.
